Europäisches Patentamt

European Patent Office

Office européen des brevets

(19)

(11) Veröffentlichungsnummer: **0 203 478**
**B1**

(12)

# EUROPÄISCHE PATENTSCHRIFT

(45) Veröffentlichungstag der Patentschrift:
08.03.89

(51) Int. Cl.⁴: **C07C 19/045**, C07C 17/38

(21) Anmeldenummer: 86106682.7

(22) Anmeldetag: 16.05.86

(54) Verfahren zur Reinigung von 1,2-Dichlorethan.

(30) Priorität: 29.05.85 DE 3519161

(43) Veröffentlichungstag der Anmeldung:
03.12.86 Patentblatt 86/49

(45) Bekanntmachung des Hinweises auf die Patenterteilung:
08.03.89 Patentblatt 89/10

(84) Benannte Vertragsstaaten:
BE CH DE FR GB IT LI NL

(56) Entgegenhaltungen:
EP-A- 0 014 305
EP-A- 0 131 932
JP-A-60 115 535

R.A. VAUCK et al.: "Grundoperationen chemischer
Verfahrenstechnik", 3. Auflage 1969. VERLAG
THEODOR STEINKOPFF, Dresden, Seiten 485-488

(73) Patentinhaber: HOECHST AKTIENGESELLSCHAFT,
Postfach 80 03 20, D-6230 Frankfurt am Main 80(DE)

(72) Erfinder: Schwarzmaier, Peter, Billerstrasse 10,
D-8261 Kastl(DE)
Erfinder: Fröhlich, Walter, Dr., Kampenwandstrasse 11,
D-8269 Burgkirchen(DE)
Erfinder: Kühn, Wenzel, Dr., Kampenwandstrasse 15,
D-8269 Burgkirchen(DE)
Erfinder: Riedl, Josef, Dr., Kantstrasse 53,
D-8269 Burgkirchen(DE)
Erfinder: Schaffelhofer, Iwo, Dr., Kettelerstrasse 2,
D-8263 Burghausen(DE)
Erfinder: Mittermaier, Erich, Spridererweg 6,
D-8261 Tüssling(DE)
Erfinder: Krumböck, Reinhard, Lohnerstrasse 40,
D-8269 Burgkirchen(DE)

## Beschreibung

Die Erfindung betrifft ein Verfahren zur Reinigung von 1,2-Dichlorethan gemäß Anspruch 1.

1,2-Dichlorethan wird in großen Mengen produziert. Der größte Teil hiervon wird thermisch gespalten, wobei Vinylchlorid und Chlorwasserstoff entstehen. Für diese thermische Spaltung muß das 1,2-Dichlorethan eine hohe Reinheit aufweisen. Üblicherweise wird nur ein Teil des 1,2-Dichlorethans thermisch gespalten, ein beträchtlicher Teil bleibt unverändert und wird nach Reinigung von den Spaltungs-Nebenprodukten wieder in die Spaltung zurückgeführt. Der umgesetzte Teil des 1,2-Dichlorethans wird hierbei ständig durch frisch hergestelltes Produkt ergänzt. Für die letztere Herstellung wird etwa je zur Hälfte die Reaktion von Ethylen mit Chlor (nachfolgend "Direktchlorierung" genannt) und die Umsetzung von Ethylen mit Chlorwasserstoff und Sauerstoff (nachlfolgend "Oxychlorierung" genannt) verwendet. Entsprechend seiner verschiedenen Herkunft enthält das 1,2-Dichlorethan unterschiedliche Verunreinigungen, die in der Regel durch drei verschiedene Reinigungsschritte entfernt werden.

1. Abdestillieren des Wassers und der Leichtsieder,
2. Abdestillieren der Hauptmenge des 1,2-Dichlorethans,
3. Abdestillieren der Restmenge des 1,2-Dichlorethans, im Sumpf verbleiben die Hochsieder.

Während die Destillationskolonnen für 1. und 2. üblicherweise bei normalem Luftdruck betrieben werden, wird bei 3. Unterdruck angewandt.

Häufig wird das im Kreis geführte 1,2-Dichlorethan vor der Destillation noch mit chemischen Mitteln (beispielsweise Chlor) vorbehandelt (siehe hierzu beispielsweise DE-C 2 416 789).

Um die Reinigung des 1,2-Dichlorethans möglichst wirtschaftlich durchzuführen, sind Verfahren erwünscht, die eine möglichst gute Ausnutzung der anfallenden beziehungsweise aufzuwendenden Energie ermöglichen. Hierzu ist aus DE-OS 2 427 045 ein Verfahren bekannt, Ethylen mit Chlor in einem umlaufenden Medium, vorzugsweise 1,2-Dichlorethan, unter Druck unterhalb der Verdampfungstemperatur des 1,2,-Dichlorethans umzusetzen und das Reaktionsgemisch in eine Zone niedrigeren Druckes überzuführen. In dieser Zone verdampft ein Teil des 1,2-Dichlorethans, wird über eine Rektifizierzone gereinigt und abgezogen, während die Restmenge des 1,2-Dichlorethans im Kreis geführt wird. Das 1,2-Dichlorethan wird dabei mit der Reaktionswärme aus der Umsetzung von Ethylen und Chlor gereinigt.

Ähnlich arbeitet ein anderes aus DE-A 2 935 884 bekanntes Verfahren, bei dem Drücke von 0,3 bis 1,3 bar und Temperaturen von 50 bis 90°C angewendet werden, wobei ein Teil des Chlors im Reaktionsprodukt gelöst wird. Vor dem Lösen wird das Reaktionsprodukt mit Ortho- oder Meta-Kresol oder deren Mono- oder Dichlorderivaten versetzt.

Ein drittes aus DE-A 3 137 513 bekanntes Verfahren führt die Umsetzung von Ethylen mit Chlor bei Temperaturen von 75 bis 200°C und Drücken von 1 bis 15 bar durch, zieht einen Teil des flüssigen Reaktionsgemisches ab und teilt diesen in zwei Ströme. Aus einem der beiden Ströme wird soviel 1,2-Dichlorethan verdampft, wie durch die Reaktion von Ethylen mit Chlor wieder nachgebildet wird. Das verdampfte 1,2-Dichlorethan wird rektifiziert, das nicht-verdampfte Produkt in die Umsetzungszone zurückgeführt. Der andere der beiden Ströme durchläuft einen Wärmetauscher. Ein Teil der darin übertragenen Wärmemenge wird zur Destillation des 1,2-Dichlorethans verwendet, wobei auf anderem Wege erzeugtes 1,2-Dichlorethan beigemischt werden kann. Höhersiedende Produkte werden aus dem Kolonnensumpf abezogen.

Die beiden erstgenannten Verfahren haben den Nachteil, daß schwerer als 1,2-Dichlorethan verdampfende Verunreinigungen im System verbleiben, was zu Schwierigkeiten führt. Teilweise müssen Fremdstoffe (Kresole oder deren Chlorierungsprodukte) zugegeben werden. Alle drei Verfahren sind nicht geeignet, wasserhaltiges 1,2-Dichlorethan aus der Oxychlorierung zu reinigen. im Prinzip ist die Zugabe von 1,2-Dichorethan anderer Herkunft beim dritten Verfahren zwar möglich, doch erfolgt diese in dem Direktchlorierungsreaktor, in welchem Wasser, wegen Korrosionsproblemen, unerwünscht ist.

Aus EP-A 131 932 ist es bekannt, 1,2-Dichlorethan in einer Destillationskolonne, in der Hochsieder abgetrennt werden, unter Einstellung des Druckes am Kopf der Kolonne, auf mehr als eine Atmosphäre (absolut) einzustellen, das aus der Kolonne abgezogene gasförmige 1,2-Dichlorethan in einem Kompressor auf einen Druck, der mindestens 0,5 kg/cm$^2$ über dem Kopfdruck der Kolonne liegt und eine Temperatur, die mindestens 7°C über der Kopftemperatur der Kolonne liegt, zu bringen und aus diesem Gas Wärme zu gewinnen. Der Druck am Kopf der Kolonne wird dabei zwischen 0,5 und 1,5 kg/cm$^2$ über dem normalen Luftdruck eingestellt und im Kompressor auf Werte zwischen 0,5 und 2,0 kg/cm$^2$ höher als dem Kopfdruck der Kolonne gebracht. Es wird davon abgeraten, den Druck in der Kolonne höher als 1,5 kg/cm$^2$ über den normalen Luftdruck ansteigen zu lassen und entsprechend die Kolonne bei erhöhter Temperatur zu betreiben, da dies die Zersetzung der hochsiedenden Substanzen im 1,2-Dichlorethan beschleunigt, wodurch Probleme bei der Reinigung dieses Stoffes entstehen.

Der Nachteil dieses Verfahrens ist die Notwendigkeit, kostenaufwendige Kompressoren zu verwenden, die in der Regel nicht direkt mit Wärmeenergie sondern mit teurerer elektrischer Energie betrieben werden.

Es wurde nun ein Verfahren gefunden, das die oben beschriebenen Nachteile nicht aufweist und ohne höhere Investitionskosten in meist vorhandenen Apparaturen mit geringen Umbaukosten durchgeführt werden kann. Dieses Verfahren zur Reinigung von 1,2-Dichlorethan, das weniger als 3 Gew.-% Stoffe enthält, die bei Atmosphärendruck einen höheren Siedepunkt als 1,2-Dichlorethan aufweisen, durch Destillation in einer ersten Kolonne unter ständiger Zufuhr von verunreinigtem 1,2-Dichlorethan, und Einführen des Sumpfproduktes dieser Kolonne in eine zweite Destillationskolonne, die unter niedrigerem Druck als die erste Kolonne arbeitet, ist dadurch gekennzeichnet, daß der Ablauf des Sumpfproduktes der ersten Kolonne so geregelt wird, daß dieses höchstens 7 Gew.-% höher als 1,2-Dichlorethan siedende Stoffe enthält, die Kopftemperatur in der ersten Kolonne auf 125 bis 180°C eingestellt wird sowie am Kopf dieser Kolonne gasförmiges 1,2-Dichlorethan abgeführt, durch einen oder mehrere Wärmetauscher geleitet wird, der/die zur Erwärmung von 1,2-Dichlorethan enthaltenden Produktströmen dient/dienen und anschließend in flüssiger Form teilweise in die genannte erste Kolonne zurückführt, teilweise als gereinigtes Produkt abgeführt und weiterverwendet wird.

Es wurde gefunden, daß im Gegensatz zu dem bekannten Stand der Technik die Kolonne, in der 1,2-Dichlorethan von höhersiedenden Substanzen abgetrennt wird, bei vergleichsweise hohen Temperaturen und entsprechenden Drücken ohne Schwierigkeiten betrieben werden kann, wenn man dafür sorgt, daß das Sumpfprodukt der Kolonne nicht mehr als höchstens 7 Gew.-%, vorzugsweise 2 bis 5 Gew.-%, höher als 1,2-Dichlorethan siedende Stoffe enthält. Das in die Kolonne eingeführte 1,2-Dichlorethan soll weniger als 3 Gew.-% höhersiedende Stoffe aufweisen, ist dies nicht der Fall, kann die Kolonne im allgemeinen nicht mehr wirtschaftlich betrieben werden. Vorzugsweise enthält das eingeführte Produkt 0,1 bis 1 Gew.-% höher als 1,2-Dichlorethan siedende Stoffe.

Das höchstens 7 Gew.-% höher als 1,2-Dichlorethan siedende Stoffe enthaltende Sumpfprodukt aus der genannten Kolonne wird in eine zweite Destillationskolonne eingeführt, die vorteilhaft bei einem Druck von 5 bis 40 kPa betrieben wird, in welcher weiteres 1,2-Dichlorethan aus dem Sumpfprodukt der ersten Kolonne abdestilliert wird. Über 40 kPa Druck gelingt die Abtrennung des 1,2-Dichlorethans nur unvollständig, unter 5 kPa Druck wird der apparative Aufwand im allgemeinen zu hoch und steht in keinem Verhältnis mehr zu dem damit zusätzlich erzielten Effekt. Vorzugsweise werden 8 bis 30 kPa angewendet.

In einer bevorzugten Ausführungsform des erfindungsgemäßen Verfahrens wird der bei einem Druck von 5 bis 40 kPa betriebenen Kolonne gasförmiges 1,2-Dichlorethan zugeführt, welches aus einer Umsetzung von Ethylen mit Chlor in Gegenwart eines Katalysators bei 40 bis 110°C stammt. Unter 40°C wird die Wärmerückgewinnung uniteressant, über 110°C nehmen die gebildeten Nebenprodukte deutlich zu, so daß Ausbeuteverluste entstehen und ein höherer Reinigungsaufwand erforderlich wird. Zwischen dem Behälter, in dem die Umsetzung stattfindet und der Kolonne ist dabei zweckmäßig eine Druckregelung eingeschaltet. Die Umsetzung des Ethylens mit Chlor wird nach bekannten Verfahren in Gegenwart katalytischer Mengen einer Lewis-Säure, beispielsweise Eisen-(III)-chlorid, durchgeführt, Vorteilhaft wird ein Katalysatorsystem angewandt, wie es in der EP 082 342-A2 beschrieben ist.

In einer weiteren vorteilhaften Ausführungsform des erfindungsgemäßen Verfahrens wird gasförmiges 1,2-Dichlorethan, das wie oben beschrieben, aus einer Umsetzung von Ethylen mit Chlor bei 40 bis 110°C in Gegenwart eines Katalysators stammt, auf den Druck verdichtet, der in der Kolonne herrscht, die mit höchstens 7 Gew.-% Hochsiedern im Sumpf betrieben wird und in diese Kolonne eingespeist.

Die Kolonne, deren Sumpfprodukt höchstens 7 Gew.-% höher als 1,2-Dichlorethan siedende Stoffe (nachfolgend "Hochsieder" genannt) enthält, wird mit einer Kopftemperatur von 125 bis 180 °C betrieben. Bei unter 125°C ist die Verwendbarkeit des aus der Kolonne gasförmig abgeführten 1,2-Dichlorethans erheblich eingeschränkt, so daß es hier vorteilhafter ist, ein solches Produkt gemäß dem Stand der Technik auf höheren Druck und höhere Temperatur zu verdichten. Wird die Kolonne mit einer Kopftemperatur über 180°C betrieben, so treten in zunehmendem Maße Schwierigkeiten durch Zersetzungsprodukte auf. Vorteilhaft wird die Kopftemperatur der Kolonne auf 130 bis 160°C, insbesondere auf 135 bis 155°C eingestellt.

Die im vorigen Absatz beschriebene Kolonne wird unter ständiger Zufuhr von verunreinigtem 1,2-Dichlorethan betrieben. Dieses kann verschiedener Herkunft sein. Beispielsweise kann es aus einer Direktchlorierung von Ethylen stammen, aus der, wenn das Reaktionsprodukt flüssig abgeführt wurde, der Katalysator wie üblich ausgewaschen und das gewaschene Rohprodukt in einer Kolonne von Wasser und leichter als 1,2-Dichlorethan siedenden Stoffen abgetrennt wurde. Es kann auch aus einer Oxychlorierung stammen und vorher, wie beschrieben, durch Abtrennen von Wasser und leichter als 1,2-Dichlorethan siedenden Stoffen gereinigt worden sein. Ferner kann das 1,2-Dichlorethan aus der thermischen Spaltung stammen, in der es nicht umgesetzt wurde und aus deren Folgeprodukten es nach Abtrennung des Chlorwasserstoffs und des gebildeten Vinylchlorids erhalten wurde. Ein solches Produkt wird zweckmäßig einer Vorreinigung, beispielsweise durch Zugabe von Chlor, unterzogen, wodurch unerwünschte Nebenprodukte chloriert und damit leichter abtrennbar gemacht werden. Eine solche Chlorierung erfolgt zweckmäßigerweise bei Temperaturen von 30 bis 60°C, da bei höheren Temperaturen auch das 1,2-Dichlorethan angegriffen wird und unerwünschte Produktverluste sowie weitere Nebenreaktionen auftreten. Hierbei ist es vorteilhaft, das auf eine im oben angegebenen Bereich liegende Temperatur gekühlte, rückgewonnene 1,2-Dichlorethan im Verhältnis 1:2 bis 6 aufzuteilen, wobei die kleinere Menge entweder der Direktchlorierung von Ethylen zugeführt wird oder, wenn dort mit einem unpassenden Katalysator gearbeitet wird, für sich mit Chlor in Gegenwart von Eisen-(III)-chlorid umgesetzt wird. Die größere Menge des zurückgewonnenen 1,2-Dichlorethans wird vorteilhaft ohne Zusatz eines Katalysators mit Chlor versetzt und nach einer bestimmten Einwirkungsdauer direkt in die Kolonne eingespeist, die bei 125 bis 180°C betrieben wird. Die Menge des zugeführten Chlors wird so bemessen, daß das Produkt vor Eintritt in die Kolonne weniger als 20 Teile Chlor je 1 000 000 Teile 1,2-Dichlorethan enthält. Bei einem Verhältnis von 1 : >6 wird im allgemeinen der Benzolgehalt im Rein-1,2-Dichlorethan zu groß. Ein Verhältnis von 1 : <2 ist zwar möglich, erfordert jedoch im allgemeinen einen zu großen Chlorierungsaufwand.

Am Kopf der mit 125 bis 180°C betriebenen Kolonne wird gasförmiges 1,2-Dichlorethan abgeführt und durch einen oder mehrere Wärmetauscher geleitet, der/die zur Erwärmung von 1,2-Dichlorethan enthaltenden Produktströmen dient/dienen. Vorteilhafte Verwendungsmöglichkeiten dieses gasförmigen 1,2-Dichlorethans sind:

a) die Heizung des Sumpfproduktes einer Kolonne, in der Wasser und/oder andere Stoffe, die bei Atmosphärendruck einen niedrigeren Siedepunkt als 1,2-Dichlorethan aufweisen, aus letzterem abdestilliert werden;

b) die Heizung des Sumpfproduktes der Kolonne, die bei einem Druck von 5 bis 40 kPa betrieben wird, und aus der 1,2-Dichlorethan möglichst vollständig von den Hochsiedern abgetrennt wird;

c) Heizung des bei der thermischen Spaltung nicht – umgesetzten, vorher chlorierten 1,2-Dichlorethans, welches anschließend direkt in die bei 125 bis 180 °C betriebene Kolonne eingespeist wird;

d) Heizung des Teilstroms des flüssigen, kondensierten und unter die Kondensationstemperatur gekühlten 1,2-Dichlorethans, das nach Durchlaufen der Wäremtauscher der unter a) bis c) genannten Prozesse oder weiterer Heizprozesse angefallen ist, bevor dieser Teilstrom als Rücklauf in die Kolonne, die bei 125 bis 180°C betrieben wird, eingespeist wird;

e) Heizung des Teilstroms an kondensiertem flüssigem 1,2-Dichlorethan, das mindestens einen Wärmetauscher für die unter a) bis c) genannten oder weitere Heizprozesse durchlaufen hat, wonach das so aufgeheizte flüssige 1,2-Dichlorethan der thermischen Spaltung zur Erzeugung von Vinylchlorid zugeführt wird.

Aus Gründen der Betriebssicherheit und um einen möglichst günstigen Energieausnutzungsverbund zu erreichen, ist es vorteilhaft, neben den Wärmetauschern, die mit gasförmigem 1,2-Dichlorethan geheizt werden und zur Heizung der Sumpfprodukte verschiedener Kolonnen dienen, noch jeweils einen zweiten Wäremaustauscher vorzusehen, der, wie üblich, mit Wasserdampf beheizt werden kann. Ferner ist es von Vorteil, die Standhöhe des flüssigen 1,2-Dichlorethans in den Wärmeaustauschern zu regulieren und mit einer Regelung des Drucks am Kopf der Kolonne, die mit 125 bis 180 °C betrieben wird, zu verbinden.

Das erfindungsgemäße Verfahren ermöglicht eine gute Ausnutzung der für die Reinigung von 1,2-Dichlorethan aufzuwendenden Energie. Ein besonderer Vorteil des Verfahrens ist, daß in meist vorhandenen Vorrichtungen ohne größere Umbauten und Investitionskosten für neue Apparaturen gearbeitet werden kann.

Nachfolgende Beispiele sollen die Erfindung näher erläutern:

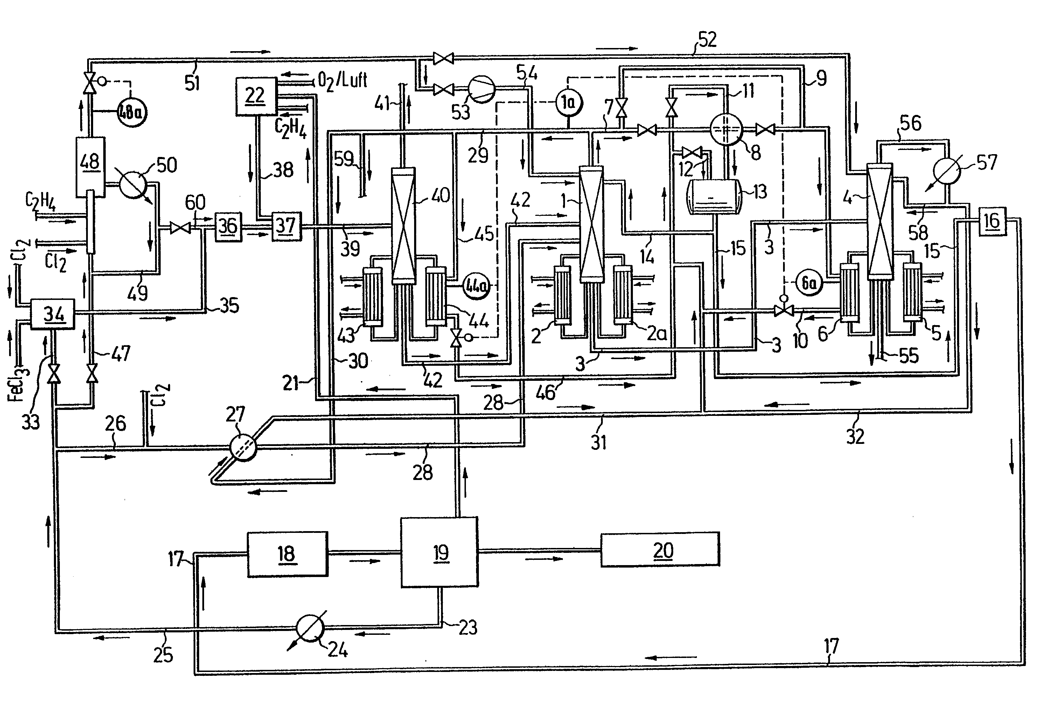

Fig. 1 zeigt schematisch eine Vorrichtung, die zur Durchführung des erfindungsgemäßen Verfahrens geeignet ist:
Eine bis 1500 kPa druckfeste Destillationskolonne (1) ist verbunden mit zwei Wärmetauschern (2) und (2a), die mit Wasserdampf betrieben werden können. Der Sumpf der Kolonne wird über die Leitung (3) in die vakuumfeste Kolonne (4) geleitet. Diese ist wiederum mit zwei Wärmetauschern verbunden, von denen der eine (5) mit Wasserdampf betrieben werden kann. In den anderen der beiden Wärmetauscher (6) kann entweder über die Leitung (7) über einen Kreuzwärmetauscher (8) oder über die Leitung (9) direkt heißes 1,2-Dichlorethangas eingeleitet werden, welches am Kopf der Kolonne (1) abgezogen wurde. Das Kondensat aus dem Wärmetauscher (6) wird entweder über die Leitungen (10) und (11) durch den Kreuzwärmetauscher (8) oder über Leitung (12) direkt in einen Vorratsbehälter (13) für Rein-1,2-Dichlorethan geleitet. Von diesem wird ein Teil über die Leitung (14) in die Kolonne (1) zurückgeführt, der Rest geht über die Leitung (15) zu einem weiteren Lagerbehälter für reines 1,2-Dichlorethan (16), aus dem über die Leitung (17) die thermische Spaltung (18) gespeist wird.

Die Produkte aus der thermischen Spaltung werden in einer hier nicht näher gezeigten Apparatekombination (19) aufgearbeitet. Das Vinylchlorid wird in einen Sammelbehälter (20) geleitet und von dort aus weiterverwendet. Der Chlorwasserstoff geht über die Leitung (21) in eine Oxychlorierungsapparatur (22), in die außerdem Ethylen und Sauerstoff in Gestalt von Luft eingespeist werden.

Das bei der thermischen Spaltung nicht-umgesetzte, verunreinigte 1,2-Dichlorethan läuft aus der Aufarbeitung (19) über die Leitung (23) zu einem Kühler (24), wo es auf etwa 40 bis 60°C abgekühlt wird. Es verläßt den Kühler über die Leitung (25) und wird in zwei Teilströme aufgeteilt. In den größeren der beiden Teilströme wird in der Leitung (26) Chlor zugegeben. Nach einer bestimmten Strecke, die zur Abreaktion des Chlors notwendig ist, durchläuft das Produkt einen Wärmetauscher (27), das aufgeheizte Produkt wird über die Leitung (28) der Kolonne (1) zugeführt. Aus dem am Kopf der Kolonne (1) abgezogenen heißen 1,2-Dichlorethangas wird ein Teilstrom abgezweigt, welcher über die Leitung (29) und nach erneuter Aufteilung über die Leitung (30) durch den Wärmetauscher (27) geführt wird, wo er das vorgereinigte Rück-1,2-Dichlorethan aus der Aufarbeitung der thermischen Spaltung (19) erwärmt. Das kondensierte flüssige 1,2-Dichlorethan wird über die Leitungen (31) und (11) bzw. (12) in den Vorratsbehälter für 1,2-Dichlorethan (13) eingeleitet.

Der kleinere Teil des gekühlten Rück-1,2-Dichlorethans aus Leitung (25) kann entweder über die Leitung (33) einem Gefäß (34) zugeführt werden, in dem es mit Chlor und einem Katalysator, beispielsweise FeCl$_3$, versetzt wird. Das aus diesem Behälter abgezogene, mit Chlor behandelte flüssige 1,2-Dichlorethan wird über die Leitung (35) und einen Wäscher (36) dem Naß-1,2-Dichlorethan-Lagerbehälter (37) zugeführt. In den gleichen Lagerbehälter wird über die Leitung (38) das ebenfalls wasserhaltige 1,2-Dichlorethan aus der Oxychlorierung (22) gegeben. Aus dem Lagerbehälter (37) wird über die Leitung (39) die Kolonne (40) gespeist. Am Kopf dieser Kolonne werden über die Leitung (41) ein azeotropes Gemisch von Wasser und 1,2-Dichlorethan sowie die Leichtsieder abgezogen. Der Sumpf der Kolonne (40) wird über die Leitung (42) in die Kolonne (1) eingespeist. Zur Erwärmung des Sumpfes der Kolonne (40) dienen zwei Wärmeaustauscher, von denen einer (43) mit Wasserdampf gespeist werden kann. In den anderen Wärmetauscher (44) wird über die Leitungen (29) und (45) heißes 1,2-Dichlorethangas, das vom Kopf der Kolonne (1) abgezogen wurde, geleitet. Das kondensierte 1,2-Dichlorethan läuft über die Leitungen (46) und entweder (11) oder (12) in den Behälter für Rein-1,2-Dichlorethan (13).

Wenn eine getrennte Chlorierung des Rück-1,2-Dichlorethans nicht erforderlich ist, wird der kleinere Teil des über die Leitung (25) ankommenden Produktes über die Leitung (47) in den Behälter (48) gegeben, in dem Ethylen mit Chlor in Gegenwart eines Katalysators in flüssigem 1,2-Dichlorethan umgesetzt wird. Das Reaktionsgemisch wird über eine Ringleitung (49) und einen Kühler (50) gekühlt.

Am Kopf des Behälters (48) kann über eine Druckregelung (48a) gasförmiges 1,2-Dichlorethan entnommen und entweder über die Leitungen (51) und (52) der Kolonne (4) zugeführt oder über die Leitung (51), den Kompressor (53) und die Leitung (54) auf entsprechenden Druck gebracht, in die Kolonne (1) eingespeist werden.

Der Sumpf der Kolonne (4) verläßt diese über die Leitung (55). Er enthält im wesentlichen die Hochsieder. Am Kopf der Kolonne (4) wird über die Leitung (56) gasförmiges 1,2-Dichlorethan abgezogen, im Kühler (57) kondensiert und teilweise über die Leitung (58) in die Kolonne (4) zurückgeleitet, der restliche Teil wird über die Leitungen (32), (31), (11) oder (12) dem Behälter für Rein-1,2-Dichlorethan (13) zugeführt. Die Standregelungen (6a) und (44a), der Wärmetauscher (6) und (44) werden von der Druckregelung (1a) der Kolonne (1) gesteuert.

Falls Bedarf besteht, können mit dem vom Kopf der Kolonne (1) abgezogenen heißen gasförmigen 1,2-Dichlorethan über die Leitungen (29) und (59) andere vorzugsweise 1,2-Dichlorethan enthaltende Stoffe geheizt werden. Ebenso kann bei Bedarf aus dem Kühlkreislauf (49) der Umsetzung von Ethylen mit Chlor ein Teil der Flüssigkeit über die Leitung (60) entnommen und in den Wäscher (36) eingespeist werden.

Beispiel 1

Es wird eine Vorrichtung gemäß Fig. 1 verwendet, wobei jedoch folgende Teile nicht benutzt werden: Leitung (33), Gefäß (34), Leitung (35), Druckregelung (48a), Leitungen (51), (52), (54) und Kompressor (53). Das im Behälter (48) aus Ethylen und Chlor erzeugte 1,2-Dichlorethan wird über die Leitung (60) in den Wäscher (36) gegeben. Der kleinere Teil des Rück-1,2-Dichlorethans wird über die Leitung (47) in den Behälter (48) eingespeist.

Mit einem Eisen-(III)-chlorid-Katalysator werden in der Direktchlorierung (Behälter 48) bei normalem Atmosphärendruck und 50°C 2,87 Gewichtsteile 1,2-Dichlorethan erzeugt. Das 1,2-Dichlorethan ist verunreinigt durch 0,098 Gew.-% Ethylchlorid als Leichtsieder und 0,205 Gew.-% 1,1,2-Trichlorethan als Schwersieder (die Prozentangaben beziehen sich auf das Rohprodukt). 1,2-Dichlorethan wird flüssig über die Leitung (60) abgezogen und im Behälter (36) mit Wasser gewaschen. Gleichzeitig werden der Direktchlorierung (48) über die Leitung (47) 1,08 Gewichtsteile bei der thermischen Spaltung nicht-umgesetztes 1,2-Dichlorethan zugeführt.

Im Behälter (22) werden aus Chlorwasserstoff, Ethylen und Luftsauerstoff 2,7 Gewichtsteile 1,2-Dichlorethan erzeugt. Dieses enthält 99,2 Gew.-% 1,2-Dichlorethan (der Rest sind niedriger und höher als 1,2-Dichlorethan siedende Stoffe) und wird in den Behälter (37) geleitet. Die dort vereinigten Produkte aus der Direktchlorierung (48) und Oxychlorierung (22) werden in der Kolonne (40) von Wasser und Leichtsiedern befreit. Der Sumpf dieser Kolonne wird durch die Wärmetauscher (43) mit Wasserdampf und (44) mit gasförmigem 1,2-Dichlorethan geheizt. Die Kolonne arbeitet unter normalem Atmosphärendruck bei einer Kopftemperatur von 75°C.

Der Sumpf der Kolonne (40) wird in die Kolonne (1) eingegeben und dort bei einer Kopftemperatur von 130°C und einem Druck am Kopf der Kolonne von 364 kPa rektifiziert. Aus dem Sumpf der Kolonne (1) werden 1,9 Gewichtsteile eines Produktes abgezogen, das 3,6 Gew.-% höher als 1,2-Dichlorethan siedende Stoffe neben 1,2-Dichlorethan enthält. Am Kopf der Kolonne (1) werden 10,65 Gew.-Teile heißes, gasförmiges 1,2-Dichlorethan abgezogen und in den oben und in die weiter unten beschriebenen Wärmetauscher geleitet, in denen es jeweils ein 1,2-Dichlorethan enthaltendes Produkt aufheizt. Der Sumpf der Kolonne (1) wird mit Wasserdampf geheizt. Das Sumpfprodukt wird in die Kolonne (4) geleitet und dort bei einer Kopftemperatur von 45°C und einem Druck am Kopf der Kolonne von 25 kPa destilliert. Der Sumpf der Kolonne (4) wird teilweise mit Wasserdampf, teilweise mit gasförmigen 1,2-Dichloethan aus der Kolonne (1) geheizt.

In einem nach bekanntem Verfahren betriebenen Spaltofen wird 1,2-Dichlorethan mit einer Reinheit von 99,6% thermisch gespalten, anschließend gekühlt und destillativ aufgearbeitet. Der hierbei aus den Spaltprodukten abgetrennte Chlorwasserstoff wird in der weiter oben beschriebenen Oxychlorierung (Behälter 22) mit Ethylen und Luft umgesetzt, das ebenfalls abgetrennte Vinylchlorid dem Lagerbehälter (20) zur weiteren Verwendung zugeführt. Das aus der Destillation als Sumpfprodukt anfallende, mit Nebenprodukten verunreinigte nicht-umgesetzte 1,2-Dichlorethan wird zunächst auf 40°C gekühlt, dann im Verhältnis 3:1 aufgeteilt. Der kleinere Teil (1.08 Gew.-Teile) der aufgeteilten Menge wird, wie oben beschrieben, der Direktchlorierung im Behälter (48) zugeführt. Der größere Teil (3,24 Gew.-

Teile) wird mit Chlor versetzt, dessen Menge so bemessen wird, daß vor Eintritt in die Kolonne (1) der Chlorgehalt des Produktes unter 0,002 Gew.-% liegt. Eine Strecke nach der Chlorzugabe wird das so behandelte 1,2-Dichlorethan in dem Wärmetauscher (27) mit heißem, gasförmigem 1,2-Dichlorethan aus der Kolonne (1) aufgeheizt und über die Leitung (28) dieser Kolonne zugeführt. Die vereinigten Ströme an kondensiertem flüssigem 1,2-Dichlorethan aus den Wärmetauschern (6), (44) und (27) sowie aus dem Kühler (57) werden, wie aus dem Schema Fig. 1 ersichtlich, über die Leitung (11) durch den Kreuzwärmetauscher (8) geleitet und in diesem mit heißem 1,2-Dichlorethangas aus Kolonne (1) geheizt. Das hierbei zur Heizung verwendete, im wesentlichen gasförmig bleibende 1,2-Dichlorethan durchläuft anschließend den Wärmetauscher (6), der den Sumpf der Kolonne (4) heizt. Das Aufteilungsverhältnis des in der thermischen Spaltung nicht-umgesetzten 1,2-Dichlorethans wird so geregelt, daß das Produkt im Behälter (13) weniger als 0,1 Gew.-% Benzol enthält. Steigt dieser Wert an, so wird der Teil des 1,2-Dichlorethans, der über die Leitung (47) in die Direktchlorierung (48) eingespeist wird, auf Kosten des Teils, der über die Leitung (26) läuft, vergrößert.

Das Rein-1,2-Dichlorethan läuft aus dem Behälter (13) mit einer Temperatur von 125 °C über die Leitung (15) in den Behälter (16).

Bei diesem Verfahren wird ein spezifischer Energieverbrauch von 131 kJ pro Kilogramm Rein-1,2-Dichlorethan festgestellt.

Vergleichsversuch A

Es wird verfahren wie in Beispiel 1 beschrieben, jedoch werden die Wärmeaustauscher (6), (44) und (27) mit Wasserdampf beheizt. Die Kolonne (1) wird mit einer Temperatur von 90°C und einem Druck am Kopf der Kolonne von 120 kPa betrieben. Das Kopfprodukt der Kolonne (1) wird bis zur Kondensation in einem Kühler (in Fig. 1 nicht gezeichnet) gekühlt und anschließend in den Behälter (13) geleitet, von wo aus es mit einer Temperatur von 70°C über die Leitung (15) in den Behälter (16) läuft. Bei diesem Verfahren wird ein spezifischer Energieverbrauch von 867 kJ/kg 1,2-Rein-Dichlorethan ermittelt.

Beispiel 2

Es wird eine Vorrichtung gemäß Fig. 1 verwendet, in der jedoch folgende Apparateteile nicht verwendet werden: die Leitungen (47), (52) und (60). In dem Behälter (48) werden bei 97°C und einem Druck von 150 kPa unter Verwendung eines eisenhaltigen Katalysators nach DE-OS 31 48 450 durch Umsetzung von Ethylen mit Chlor 2,86 Teile 1,2-Dichlorethan erzeugt, welches mit 0,005 Gew.-% Ethylchlorid als Leichtsieder und 0,103 Gew.-% 1,1,2-Trichlorethan als Hochsieder verunreinigt ist. Dieses Produkt wird gasförmig über die Leitung (51) dem Turboverdichter (53) zugeführt, in dem es von 150 kPa Druck auf 450 kPa Druck verdichtet und auf eine Temperatur von 140°C erwärmt, über die Leitung (54) in die Kolonne (1) eingespeist wird.

Die thermische Spaltung des 1,2-Dichlorethans und die Aufarbeitung der Spaltprodukte erfolgt wie in Beispiel 1 beschrieben. Das bei der Spaltung nicht-umgesetzte, verunreinigte 1,2-Dichlorethan wird auf 40°C gekühlt und wiederum im Verhältnis 1 : 3 aufgeteilt. Der kleinere Teil (1,08 Gew.-Teile) fließt über die Leitung (33) in den Behälter (34), wo es in Gegenwart von Eisen-(III)-chlorid mit Chlor versetzt wird. Das chlorierte Produkt läuft über die Leitung (35) in die Wasserwäsche (36) und von dort in den Lagerbehälter (37), wo es mit dem aus der Oxychlorierung (22) über die Leitung (38) ankommenden 1,2-Dichlorethan-Strom vereinigt wird. Die vereinigten Ströme werden dann, wie in Beispiel 1 beschrieben, in der Kolonne (40) von Wasser und Leichtsiedern getrennt, der Sumpf dieser Kolonne in die Kolonne (1) eingespeist.

Der größere Teil des in der thermischen Spaltung nicht-umgesetzten, verunreinigten 1,2-Dichlorethans (3,24 Gew.-Teile) werden, wie in Beispiel 1 beschrieben, mit Chlor versetzt, geheizt und in die Kolonne (1) eingespeist.

Die Kolonne (1) wird mit einer Kopftemperatur von 135°C und einem Druck am Kopf der Kolonne von 400 kPa betrieben. Am Kopf der Kolonne werden 10,65 Gew.-Teile 1,2-Dichlorethan gasförmig abgezogen und, wie in Beispiel 1 beschrieben, in die Wärmeaustauscher (27), (44) sowie über den Kreuzwärmetauscher (8) in den Wärmetauscher (6) geleitet. Die Rückführung des flüssigen, kondensierten 1,2-Dichlorethans aus den Wärmetauschern in den Behälter (13) erfolgt wie in Beispiel 1 beschrieben.

Aus dem Sumpf der Kolonne (1) werden 1,9 Gew.-Teile eines Produktes abgezogen, das 3,5 Gew.-% höher als 1,2-Dichlorethan siedende Stoffe neben 1,2-Dichlorethan enthält. Dieses Produkt wird der Kolonne (4) zugeführt und dort mit einer Kopftemperatur von 40°C und einem Druck am Kopf der Kolonne von 20 kPa destilliert. Sumpf- und Kopfprodukt dieser Kolonne werden behandelt wie bei Fig. 1 beschrieben.

Es wird ein spezifischer Energieverbrauch von 35 kJ/kg Rein-1,2-Dichlorethan festgestellt.

Beispiel 3

Es wird eine Vorrichtung verwendet, wie in Fig. 1 beschrieben, wobei folgende Apparateteile nicht benutzt werden: die Leitungen (47) und (60), der Kompressor (53) und die Leitung (54). Mit einem eisenhaltigen Katalysator nach DE-OS 3 148 450 werden im Behälter (48) durch Umsetzung von Ethylen mit Chlor bei 97°C und einem Druck von 150 kPa 2,86 Gew.-Teile 1,2-Dichlorethan erzeugt. Das über die Leitung (51) gasförmig abgeführte 1,2-Dichlorethan ist durch 0,0049 Gew.-% Ethylchlorid als Leichtsieder und 0,097 Gew.-% 1,1,2-Trichlorethan als Hochsieder verunreinigt (alle Gewichtsprozent-Angaben bezogen auf das verunreinigte Produkt). Nach Passieren eines Druckminderventils wird das in Leitung (51) herangeführte 1,2-Dichlorethan über die Leitung (52) in die Kolonne (4) eingeleitet, welche mit einer Kopftemperatur von 50°C und einem Druck am Kopf der Kolonne von 30 kPa betrieben wird.

Die Kolonne (1) wird mit einer Kopftemperatur von 160°C und mit einem Druck am Kopf der Kolonne von 650 kPa betrieben. Am Kopf der Kolonne (1) werden 6,9 Gew.-Teile gasförmiges 1,2-Dichlorethan abgezogen und weiterverwendet wie in Beispiel 1 beschrieben. Aus dem Sumpf der Kolonne (1) werden 1,9 Gew.-Teile eines Produktes, das 3,4 Gew.-% höher als 1,2-Dichlorethan siedende Stoffe neben 1,2-Dichlorethan enthält, abgezogen und in die Kolonne (4) eingeführt.

Das in der thermischen Spaltung nicht-umgesetzte, verunreinigte 1,2-Dichlorethan wird weiterbehandelt wie in Beispiel 2 beschrieben. Im übrigen wird in Beispiel 3 so verfahren, wie aus Beispiel 1 bzw. der Erklärung zu Fig. 1 ersichtlich.

Es wird ein spezifischer Energieverbrauch von 101 kJ/kg Rein-1,2-Dichlorethan festgestellt.

In allen Beispielen sowie im Vergleichsversuch beziehen sich alle Angaben von Gewichtsteilen auf die gleiche Zeiteinheit.

**Patentansprüche**

1. Verfahren zur Reinigung von 1,2-Dichlorethan, das weniger als 3 Gew.-% Stoffe enthält, die bei Atmosphärendruck einen höheren Siedepunkt als 1,2-Dichlorethan aufweisen, durch Destillation in einer ersten Kolonne, unter ständiger Zufuhr von verunreinigtem 1,2-Dichlorethan, Einführen des Sumpfproduktes dieser Kolonne in eine zweite Destillationskolonne, die unter niedrigerem Druck als die erste Kolonne arbeitet, dadurch gekennzeichnet, daß der Ablauf des Sumpfproduktes der ersten Kolonne so geregelt wird, daß dieses höchstens 7 Gew.-% höher als 1,2-Dichlorethan siedende Stoffe enthält, die Kopftemperatur in der ersten Kolonne auf 125 bis 180°C eingestellt wird sowie am Kopf dieser Kolonne gasförmiges 1,2-Dichlorethan abgeführt, durch einen oder mehrere Wärmetauscher geleitet wird, der/die zur Erwärmung von 1,2-Dichlorethan enthaltenden Produktströmen dient/dienen, und anschließend in flüssiger Form teilweise in die genannte erste Kolonne zurückgeführt, teilweise als gereinigtes Produkt abgeführt und weiterverwendet wird.

2. Verfahren nach Anspruch 1, dadurch gekennzeichnet, daß die zweite Destillationskolonne, in die das höchstens 7 Gew.-% höher als 1,2-Dichlorethan siedende Stoffe enthaltende Sumpfprodukt der ersten Kolonne eingeführt wird, bei einem Druck von 5 bis 40 kPa betrieben wird, wobei gasförmiges 1,2-Dichlorethan aus einer Umsetzung von Ethylen mit Chlor in Gegenwart eines Katalysators bei 40 bis 110°C zugeführt wird.

3. Verfahren nach Anspruch 1, dadurch gekennzeichnet, daß gasförmiges 1,2-Dichlorethan aus einer Umsetzung von Ethylen mit Chlor in Gegenwart eines Katalysators bei 40 bis 110°C auf den in der in Anspruch 1 genannten ersten Kolonne herrschenden Druck verdichtet und in diese Kolonne eingespeist wird.

4. Verfahren nach Anspruch 2, dadurch gekennzeichnet, daß das Sumpfprodukt der zweiten, bei einem Druck von 5 bis 40 kPa betriebenen Destillationskolonne in einem Wärmetauscher mit gasförmigem 1,2-Dichlorethan aus der in Anspruch 1 beschriebenen ersten Kolonne geheizt wird.

5. Verfahren nach Anspruch 1, dadurch gekennzeichnet, daß das Sumpfprodukt einer Kolonne, in der aus verunreinigtem 1,2-Dichlorethan Wasser und/oder andere Stoffe, die bei Atmosphärendruck einen niedrigeren Siedepunkt als 1,2-Dichlorethan aufweisen, abdestilliert werden, in einem Wärmetauscher mit gasförmigem 1,2-Dichlorethan aus der in Anspruch 1 beschriebenen ersten Kolonne geheizt wird.

6. Verfahren nach einem oder mehreren der Ansprüche 1, 4 und 5, dadurch gekennzeichnet, daß das gasförmige 1,2-Dichlorethan aus der in Anspruch 1 beschriebenen ersten Kolonne, gegebenenfalls nach Durchlaufen von einem oder mehreren Wärmetauschern, die zur Erwärmung des 1,2-Dichlorethan enthaltenden Sumpfproduktes anderer Destillationskolonnen dienen, zumindest teilweise einen weiteren Wärmetauscher durchläuft, in dem das mit Chlor versetzte Sumpfprodukt aus einer Kolonne, in der Vinylchlorid von 1,2-Dichlorethan abdestilliert worden ist, geheizt wird, wonach dieses Sumpfprodukt in die in Anspruch 1 beschriebene erste Kolonne eingeführt wird.

7. Verfahren nach einem oder mehreren der Ansprüche 1 bis 6, dadurch gekennzeichnet, daß 1,2-Dichlorethan, das gasförmig am Kopf der in Anspruch 1 genannten ersten Kolonne abgezogen wurde und mindestens einen Wärmetauscher durchlaufen hat, wobei es in flüssigen Zustand übergeführt wurde, mit gasförmigem 1,2-Dichlorethan aus der genannten ersten Kolonne erwärmt und in diese Kolonne zurückgeführt wird.

8. Verfahren nach einem oder mehreren der Ansprüche 1 bis 7, dadurch gekennzeichnet, daß 1,2-Dichlorethan, das am Kopf der in Anspruch 1 genannten ersten Kolonne abgezogen wurde und mindestens einen Wärmetauscher durchlaufen hat, wobei es in flüssigen Zustand übergeführt wurde, mit gasförmigem 1,2-Dichlorethan aus der genannten ersten Kolonne erwärmt und einer thermischen Spaltung zur Erzeugung von Vinylchlorid zugeführt wird.

**Claims**

1. A process for the purification of 1,2-dichloroethane containing less than 3% by weight of substances having a boiling point under atmospheric pressure higher than 1,2-dichloroethane, by distillation in a first column with continuous admission of impure 1,2-dichloroethane, and by the introduction of the bottom product from this column into a second distillation column operating under a lower pressure than the first column, characterised by regulating the discharge of the bottom product from the first column in such a way that this product contains not more than 7% by weight of substances boiling above 1,2-dichloroethane, adjusting the head temperature in the first column to 125 to 180°C and taking off gaseous 1,2-dichloroethane at the head of this column, passing it through one or more heat exchangers which serve(s) to heat up product streams containing 1,2-dichloroethane and then recycling part of it in liquid form to the first column mentioned and removing part of it as a purified product and using the latter for further purposes.

2. The process as claimed in claim 1, characterised in that the second distillation column, into which is introduced the bottom product from the first column, containing not more than 7% by weight of substances boiling above 1,2-dichloroethane, is operated at a pressure of 5 to 40 kPa, and is fed with gaseous 1,2-dichloroethane from the reaction of ehtylene with chlorine in the presence of a catalyst at 40 to 110°C.

3. The process as claimed in claim 1, characterised in that gaseous 1,2-dichloroethane from the reaction of ethylene with chlorine in the presence of a catalyst at 40 to 110°C is compressed to the pressure prevailing in the first column mentioned in claim 1 and is fed to this column.

4. The process as claimed in claim 2, characterised in that the bottom product from the second distillation column, operated at a pressure of 5 to 40 kPa, is heated in a heat exchanger with gaseous 1,2-dichloroethane from the first column described in claim 1.

5. The process as claimed in claim 1, characterised in that the bottom product from a column in which water and/or other substances having a boiling point lower than 1,2-dichloroethane under atmospheric pressure are removed by distillation from impure 1,2-dichloroethane, is heated in a heat exchanger with gaseous 1,2-dichloroethane from the first column described in claim 1.

6. The process as claimed in one or more of claims 1, 4 and 5, characterised in that the gaseous 1,2-dichloroethane from the first column described in claim 1, if appropriate after passing through one or more heat exchangers serving to heat up the bottom procuct, containing 1,2-dichloroethane, from other distillation columns, is passed, at least in part, through a further heat exchanger in which the chlorine-treated bottom product from a column in which vinyl chloride has been removed from 1,2-dichloroethane by distillation, is heated, after which this bottom product is fed to the first column described in claim 1.

7. The process as claimed in one or more of claims 1 to 6, characterised in that 1,2-dichloroethane which has been taken off in the form of gas at the head of the first column mentioned in claim 1 and which has passed through at least one heat exchanger, in the course of which it has been converted into the liquid state, is heated by means of gaseous 1,2-dichloroethane from the first column mentioned and is recycled to this column.

8. The process as claimed in one or more of claims 1 to 7, characterised in that 1,2-dichloroethane which has been taken off at the head of the first column mentioned in claim 1 and which has passed through at least one heat exchanger, in the course of which it has been converted into the liquid state, is heated by means of gaseous 1,2-dichloroethane from the first column mentioned and is fed to a thermal cracking reaction for the production of vinyl chloride.

**Revendications**

1. Procédé de purification d'un dichloro-1,2 éthane, contenant moins de 3% en poids de substances présentant sous la pression atmosphérique un point d'ébullition supérieur à celui du dichloro-1,2 éthane, par distillation dans une première colonne, avec apport continu de dichloro-1,2 éthane contaminé, introduction du produit de queue de cette colonne dans une deuxième colonne de distillation qui travaille sous une pression plus faible que la première colonne, caractérisé en ce que l'évacuation du produit de queue de la première colonne est ajusté de façon qu'il contienne au plus 7% en poids de substances ayant un point d'ébullition supérieur à celui du dichloro-1,2 éthane, on ajuste la température de tête de la première colonne à 125–180°C et on évacue en tête de cette colonne le dichloro-1,2 éthane gazeux, on le fait passer par un ou plusieurs échangeurs de chaleur qui servent à chauffer les courants de produits contenant le dichloro-1,2 éthane, puis on le renvoie en partie, sous forme liquide, dans ladite première colonne et, en partie, on l'évacue et on le réutilise sous forme d'un produit purifié.

2. Procédé selon la revendication 1, caractérisé en ce que la deuxième colonne de distillation, dans laquelle est introduit le produit de queue de la première colonne, lequel contient au plus 7% en poids de substances ayant un point d'ébullition supérieur à celui du dichloro-1,2 éthane, est exploitée à une pression de 5 à 40 kPa, du dichloro-1,2 éthane gazeux étant amené à partir d'une réaction de l'éthylène sur du chlore en présence d'un catalyseur à 40–110°C.

3. Procédé selon la revendication 1, caractérisé en ce que le dichloro-1,2 éthane gazeux provenant d'une réaction de l'éthylène sur du chlore en présence d'un catalyseur à une température de 40 à 110°C est comprimé à la pression régnant dans la première colonne mentionnée dans la revendication 1, et est introduit dans cette colonne.

4. Procédé selon la revendication 2, caractérisé en ce que le produit de queue de la deuxième colonne de distillation, exploitée à une pression de 5 à 40 kPa, est chauffé dans un échangeur de chaleur à l'aide du dichloro-1,2 éthane gazeux provenant de la première colonne décrite dans la revendication 1.

5. Procédé selon la revendication 1, caractérisé en ce que le produit de queue d'une colonne dans laquelle on chasse par distillation, du dichloro-1,2 éthane contaminé, de l'eau et/ou d'autres substances qui présentent à la pression atmosphérique un point d'ébullition inférieur à celui du dichloro-1,2 éthane, est chauffé dans un échangeur de chaleur à l'aide du dichloro-1,2 éthane gazeux provenant de la première colonne décrite dans la revendication 1.

6. Procédé selon l'une ou plusieurs des revendications 1, 4 ou 5, caractérisé en ce que le dichloro-1,2 éthane gazeux provenant de la première colonne décrite dans la revendication 1 traverse, éventuellement après avoir traversé un ou plusieurs échangeurs de chaleur qui servent à chauffer le produit de queue — contenant du dichloro-1,2 éthane — d'autres colonnes de distillation, au moins partiellement un échangeur de chaleur supplémentaire, dans lequel est chauffé le produit de queue, additionné de chlore, provenant d'une colonne dans laquelle le chlorure de vinyle a été chassé par distillation du dichloro-1,2 éthane, ce aprés quoi le produit de queue est introduit dans la première colonne décrite dans la revendication 1.

7. Procédé selon l'une ou plusieurs des revendications 1 à 6, caractérisé en ce que le dichloro-1,2 éthane qui avait été soutiré sous forme gazeuse en tête de la première colonne mentionnée dans la revendication 1 et a traversé au moins un échangeur de chaleur, ou il était passé à l'état liquide, est chauffé à l'aide du dichloro-1,2 éthane gazeux provenant de le première colonne mentionnée et est renvoyé dans cette colonne.

8. Procédé selon l'une ou plusieurs des revendications 1 à 7, caractérisé en ce que le dichloro-1,2 éthane qui avait été soutiré en tête de la première colonne mentionnée dans la revendication 1 et a traversé au moins un échangeur de chaleur, en y passant à l'état liquide, est chauffé à l'aide du dichloro-1,2 éthane gazeux provenant de la première colonne mentionnée et est envoyé à une unité de dissociation thermique pour produire du chlorure de vinyle.